# FASCICULE DE BREVET EUROPEEN

(11) **EP 1 028 934 B1**
(45) Date de publication et mention de la délivrance du brevet: **22.09.2004**
(21) Numéro de dépôt: 98955536.2
(22) Date de dépôt: 29.10.1998
(51) Int. Cl.: C07B 63/00, C02F 1/04, C07D 301/12, C07D 301/32

(54) **PROCEDE POUR SEPARER UN COMPOSE ORGANIQUE D'UN MILIEU AQUEUX**
VERFAHREN ZUR ABTRENNUNG EINER ORGANISCHEN VERBINDUNG AUS EINEM WÄSSERIGEN MEDIUM
METHOD FOR SEPARATING AN ORGANIC COMPOUND FROM AN AQUEOUS MEDIUM

(30) Priorité: 07.11.1997 BE 9700896
(43) Date de publication de la demande: 23.08.2000
(73) Titulaire: SOLVAY (Société Anonyme), 1050 Bruxelles (BE)
(72) Inventeur: GOSSELIN, Benoît, B-1421 Ophain (BE); STREBELLE, Michel, B-1050 Bruxelles (BE)
(74) Mandataire: Vande Gucht, Anne
(86) Numéro de dépôt international: PCT/EP1998/007002
(87) Numéro de publication internationale: WO 1999/024382

(56) Documents cités:
- US-A- 4 562 264
- US-A- 4 977 285
- US-A- 5 340 446
- DATABASE WPI Section Ch, Week 7609 Derwent Publications Ltd., London, GB; Class D15, AN 76-15838X XP002071629 & JP 51 005269 A (TOKUYAMA SODA KK)

## Description

La présente invention se rapporte à un procédé de fabrication d'un composé organique, plus particulièrement à un procédé de traitement des effluents obtenus après la séparation du composé organique du milieu réactionnel.

Il est connu, notamment par la demande de brevet EP-A-100119, de transformer un composé oléfinique (c'est-à-dire un composé organique comportant. au moins une double liaison carbone-carbone) en l'oxirane correspondant par réaction avec du peroxyde d'hydrogène dans un milieu liquide contenant de l'eau. Ce procédé permet par exemple de synthétiser du 1,2-époxypropane (oxyde de propylène) ou du 1,2-époxy-3-chloropropane (épichlorhydrine) au départ, respectivement, de propylène ou de chlorure d'allyle.

Dans ce procédé connu, le mélange de produits réactionnels obtenu à la sortie du réacteur d'époxydation contient l'oxirane, de l'eau, divers sous-produits de réaction et éventuellement des réactifs non convertis ainsi que, le plus souvent, un diluant (par exemple du méthanol ou de l'acétone). Parmi les sous-produits, on retrouve des produits qui sont formés par réaction entre l'oxirane et de l'eau ou éventuellement le diluant. Par exemple, lorsque ce procédé est appliqué à la synthèse d'épichlorhydrine par réaction entre du chlorure d'allyle et du peroxyde d'hydrogène dans du méthanol et de l'eau, l'épichlorhydrine et l'eau (ou le méthanol) peuvent former, dans les conditions habituelles d'époxydation, des quantités notables de 1-chloro-3-méthoxypropan-2-ol, de 1-chloro-2-méthoxypropan-3-ol, de 1,3-dichloropropan-2-ol, de 2,3-dichloropropanol et de 1-chloro-2,3-dihydroxypropane. Au départ de propylène, on observe la formation de propylène glycol ainsi que de 1-méthoxypropan-2-ol et de 2-méthoxypropan-1-ol. Ces sous-produits sont solubles dans l'eau et se retrouvent dès lors dans l'effluent aqueux qui est recueilli après la séparation de l'oxirane du milieu réactionnel. Certains des sous-produits solubles dans l'eau (en particulier le 1-chloro-3-méthoxypropan-2-ol et le 1,3-dichloropropan-2-ol) forment des azéotropes avec l'eau. On ne peut par conséquent pas les séparer aisément par simple distillation ou par stripping. En outre, ces sous-produits posent des problèmes de rejet car ils contribuent à la demande chimique en oxygène et, le cas échéant, à la présence de composés halogénés indésirables.

Le document US 4977285 divulgue une réaction d'époxydation du propylène (oxiranne) suivi d'une séparation par ajout d'un alcool (solvant organique) et puis une distillation. Il semblerait que ce procédé se déroule en l'absence d'eau et en présence d'un alcool comme solvant organique qui est miscible à l'eau.

Le document Database WPI Section Ch, Week 7609 Derwent Publications Ltd., London, GB ; Class D15, AN 76-15838X XP002071629 & JP 51005269 A divulgue un procédé de séparation d'une solution aqueuse contenant des composés organiques par distillation. Ce document ne divulgue pas la nature des composés organiques et ne suggère pas l'utilisation d'un solvant organique pour extraire des composés organiques.

Le document US 4562264 divulgue la séparation des produits de synthèse des anhydrides carboxyliques cycliques caractérisé par le fait que l'on ajoute un solvant aromatique dans l'effluent en vue de la séparation.

Le document US 541 2122 divulgue un procédé de synthèse catalytique d'un oxyde d'hexène en réagissant l'hexène avec du H₂O₂ et du méthanol comme solvant. Ce document indique que l'oxiranne formé peut être séparé par une extraction liquide-liquide mais ne donne pas de détails.

L'invention a pour objet un procédé simple de fabrication d'un composé organique dans un milieu liquide contenant de l'eau, qui permette d'éliminer les sous-produits solubles dans l'eau de manière aisée et avec une efficacité élevée et de réduire ainsi les problèmes de rejet.

L'invention concerne dès lors un procédé de fabrication d'un oxiranne par réaction entre un composé oléfinique et le peroxyde d'hydrogène dans un milieu liquide contenant de l'eau, selon lequel on recueille un mélange de produits réactionnels comprenant l'oxiranne, de l'eau et des sous-produits hydroxylés formés par ouverture du cycle de l'oxiranne, on sépare au moins une partie de l'oxiranne du mélange de produits réactionnels, on recueille un effluent contenant de l'eau et des sous-produits, on ajoute à l'effluent un solvant organique et on soumet le mélange contenant l'effluent et le solvant à un traitement de distillation, le solvant étant capable d'extraire les sous-produits de l'effluent aqueux, et présentant une très faible miscibilité avec l'eau de manière à ce qu'il se forme en tête de distillation deux phases liquides.

Le solvant organique mis en oeuvre dans le procédé selon l'invention a pour fonction d'extraire les sous-produits de l'eau et de pouvoir les éliminer par distillation azéotropique.

Le procédé selon l'invention est appliqué aux sous-produits hydroxylés formés par ouverture du cycle époxyde. Les meilleurs résultats sont obtenus lorsque les sous-produits contiennent en outre un ou plusieurs groupements halogénés. Le procédé s'applique particulièrement bien pour éliminer des sous-produits tels que des diols et/ou leurs dérivés monoalkyléthers.

Dans le procédé selon l'invention, on peut recueillir après distillation de l'effluent, d'une part, en tête de distillation, une première phase liquide contenant le solvant et une deuxième phase liquide contenant de l'eau épurée en sous-produits et d'éventuelles traces de solvant, et d'autre part, en pied de distillation, un mélange de solvant et de sous-produits. Les deux phases liquides distinctes recueillies en tête de distillation peuvent être séparées selon les méthodes classiques de séparation telles que la décantation. Ainsi, on récupère, d'une part, la première phase liquide contenant le solvant que l'on peut recycler à la distillation, tel quel ou après l'avoir soumis à un traitement d'épuration. D'autre part, on récupère la deuxième phase liquide, contenant l'eau épurée en sous-produits et d'éventuelles traces de solvant, que l'on peut, éventuellement, soumettre à un stripping afin de récupérer les éventuelles traces de solvant que l'on peut recycler à la distillation. Ensuite; on peut également soumettre le pied de distillation, qui contient un mélange de solvant et de sous-produits, à une évaporation, éventuellement sous vide, afin de récupérer le solvant à l'état épuré et de le recycler à la distillation.

Le solvant peut contenir un ou plusieurs composés. Un solvant substantiellement stable et inerte chimiquement vis-à-vis des constituants de l'effluent aqueux dans les conditions de distillation, ainsi que, le cas échéant, dans les étapes ultérieures convient particulièrement bien.

Des solvants qui donnent de bons résultats sont ceux dont le poids spécifique diffère de celui de la phase liquide contenant de l'eau épurée recueillie en tête de distillation d'au moins 0,02 g/cm³, en particulier d'au moins 0,04 g/cm³. Les meilleurs résultats sont obtenus lorsque ces poids spécifiques diffèrent d'au moins 0,05 g/cm³.

Il peut s'avérer intéressant d'utiliser un solvant dont le point d'ébullition est bas par rapport aux sous-produits précités. Ceci permet, en effet, de soumettre le pied de distillation à une séparation du solvant des sous-produits par évaporation, éventuellement sous vide, de purifier ainsi le solvant et de le recycler à la distillation. On utilise habituellement des solvants dont le point d'ébullition diffère de celui des sous-produits solubles dans l'eau d'au moins 5 °C, en particulier d'au moins 10 °C. Les meilleurs résultats sont obtenus lorsque ces points d'ébullition diffèrent d'au moins 15 °C.

Des composés qui peuvent être utilisés comme solvant dans le procédé selon l'invention sont les dérivés organiques aliphatiques ou aromatiques pouvant inclure des atomes tels que l'oxygène et/ou un halogène, ainsi que leurs mélanges. On peut citer à titre d'exemples les hydrocarbures aromatiques alkylés portant un ou plusieurs groupes alkyles contenant de 1 à 4 atomes de carbone comme le toluène, le xylène, le mésitylène, l'éthylbenzène et le butylbenzène. Le xylène est particulièrement préféré. Par xylène, on entend désigner aussi bien chacun des isomères (ortho, meta, para) que leurs mélanges. On peut également citer les hydrocarbures aliphatiques saturés contenant de 5 à 12 atomes de carbone comme le pentane, l'hexane, l'octane et le décane, ainsi que des hydrocarbures aliphatiques cycliques comme la décaline.

Le procédé de distillation selon l'invention est effectué selon les méthodes classiques de distillation azéotropique. Avantageusement, on utilise comme appareillage de distillation une colonne de distillation azéotropique.

La pression à laquelle la distillation est effectuée n'est pas critique. La distillation est généralement réalisée à une pression qui peut varier d'une pression subatmosphérique à 7600 mm Hg. La pression est avantageusement au moins égale à 0,5 mm Hg. Elle est avantageusement au plus égale à 3800 mm Hg.

Les températures en tête et en pied de distillation dépendent du solvant mis en oeuvre et de la pression appliquée. En pratique, à pression atmosphérique, la température en tête de distillation est d'au moins 30 °C et inférieure à 100 °C.

Le rapport pondéral entre le solvant et l'effluent dépend du solvant mis en oeuvre et de l'appareillage de distillation utilisé. En pratique, le rapport pondéral entre le solvant et l'effluent est généralement d'au moins 0,01. De préférence, il est d'au moins 0,1. Ce rapport ne dépasse pas habituellement 5. Le plus souvent, il ne dépasse pas 1. De bons résultats ont été obtenus avec un rapport de 0,1 à 1.

L'effluent contient généralement au moins 1 % en poids de sous-produits, en particulier au moins 5 % en poids. Le plus souvent, l'effluent contient au moins 10 % en poids de sous-produits. La concentration en sous-produits ne dépasse pas, généralement, 50 % en poids de l'effluent, en particulier pas 30 % en poids. L'effluent contient le plus souvent moins de 20 % en poids de sous-produits.

L'oxirane qui peut être préparé par le procédé selon l'invention est un composé organique répondant à la formule générale :

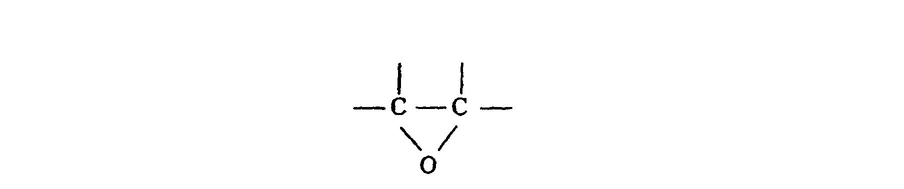

L'oxirane contient généralement de 2 à 20 atomes de carbone, de préférence de 3 à 10 atomes de carbone. Il peut renfermer des atomes d'halogène, en particulier de chlore. Un composé oléfinique qui convient bien dans le procédé selon l'invention est le chlorure d'allyle. Les composés oléfiniques peuvent également être choisis parmi les alpha-oléfines. On peut citer à titre d'exemples le propylène, le 1-octène et le 1-décène. Le propylène convient bien. Un oxirane qui peut être préparé de manière avantageuse par le procédé selon l'invention est l'épichlorhydrine. On peut également fabriquer de l'oxyde de propylène.

Dans le procédé selon l'invention, l'étape de séparation d'au moins une partie du composé organique du mélange de produits réactionnels peut être réalisée en mettant ce mélange en contact avec un liquide organique d'extraction de manière à obtenir deux phases liquides distinctes, à savoir, d'une part, un extrait organique contenant la majorité de la quantité d'oxirane produit, et, d'autre part, un raffinat aqueux contenant l'eau et les sous-produits solubles dans l'eau.

Le procédé selon l'invention s'est révélé très avantageux pour préparer le 1,2-époxy-3-chloropropane par réaction entre le chlorure d'allyle et du peroxyde d'hydrogène. Il convient également pour la préparation du 1,2-époxypropane par réaction entre le propylène et du peroxyde d'hydrogène.

Les exemples qui suivent sont destinés à illustrer la présente invention sans toutefois en limiter la portée.

### Exemple 1 (conforme à l'invention)

Un effluent aqueux sortant de la synthèse d'épichlorhydrine au départ de chlorure d'allyle et de peroxyde d'hydrogène qui contient 59,5 g/kg de 1-chloro-3-méthoxypropan-2-ol et 2,8 g/kg de 1,3-dichloropropan-2-ol a été mis en contact avec 135 g de xylène/kg d'effluent à traiter. Ce mélange a été soumis à une distillation dans une colonne de Vigreux de 1 m de hauteur. La température mesurée en tête de colonne de distillation a été de 45-49 °C et la pression absolue dans la colonne a été de 100 mm Hg. Après séparation des phases en tête de distillation, on a récupéré une phase aqueuse épurée qui représente 84,5 % de l'effluent aqueux mis en oeuvre et qui a été analysée. Le tableau 1 mentionne le taux de récupération de chacun des deux sous-produits. Le taux de récupération est le rapport (exprimé en %) entre le poids du sous-produit en question présent dans la phase aqueuse épurée et le poids de ce sous-produit présent dans l'effluent aqueux avant distillation.

**TABLEAU I**

| | |
|---|---|
| 1-chloro-3-méthoxypropan-2-ol | 1,8 |
| 1,3-dichloropropan-2-ol | 0,3 |

### Exemple 2 (non conforme à l'invention)

Le même effluent aqueux que dans l'exemple 1, mis à part qu'il contient 96,1 g/kg de 1-chloro-3-méthoxypropan-2-ol et 4,2 g/kg de 1,3-dichloropropan-2-ol, a été soumis à une distillation dans le même appareillage que dans l'exemple 1 mais sans addition de xylène. La température mesurée en tête de colonne de distillation a été de 20-30 °C et la pression absolue dans la colonne a été au départ de 30 mm Hg et est ensuite descendue à 1 mm Hg. Après distillation, on a récupéré une phase aqueuse qui représente 70,7 % de l'effluent aqueux mis en oeuvre et qui a été analysée. Le tableau II mentionne le taux de récupération de chacun des deux sous-produits.

**TABLEAU II**

| | |
|---|---|
| 1-chloro-3-méthoxypropan-2-ol | 52,4 |
| 1,3-dichloropropan-2-ol | 65,1 |

## Revendications

1. Procédé de fabrication d'un oxirane par réaction entre un composé oléfinique et le peroxyde d'hydrogène dans un milieu liquide contenant de l'eau, selon lequel on recueille un mélange de produits réactionnels comprenant l'oxirane, de l'eau et des sous-produits hydroxylés formés par ouverture du cycle de l'oxirane, on sépare au moins une partie de l'oxirane du mélange de produits réactionnels, on recueille un effluent contenant de l'eau et les sous-produits, on ajoute à l'effluent un solvant organique et on soumet le mélange contenant l'effluent et le solvant à un traitement de distillation, le solvant organique étant capable d'extraire les sous-produits de l'effluent aqueux, et présentant une très faible miscibilité avec l'eau de manière à ce qu'il se forme en tête de distillation deux phases liquides.

2. Procédé selon la revendication 1, dans lequel les sous-produits contiennent en outre un ou plusieurs groupements halogénés.

3. Procédé selon la revendication 1 ou 2, dans lequel on recueille après distillation, d'une part, en tête de distillation, une première phase liquide contenant le solvant et une deuxième phase liquide contenant de l'eau épurée en sous-produits et d'éventuelles traces de solvant, et d'autre part, en pied de distillation, un mélange de solvant et de sous-produits, on sépare les deux phases liquides recueillies en tête de distillation, et on recycle la première phase liquide contenant le solvant à la distillation.

4. Procédé selon la revendication 3, dans lequel le poids spécifique du solvant diffère de celui de la phase liquide contenant de l'eau épurée recueillie en tête de distillation d'au moins 0,04 g/cm³.

5. Procédé selon l'une quelconque des revendications 1 à 4, dans lequel le point d'ébullition du solvant diffère de celui des sous-produits d'au moins 5 °C.

6. Procédé selon l'une quelconque des revendications 1 à 5, dans lequel le solvant est choisi parmi les dérivés organiques aliphatiques ou aromatiques pouvant inclure des atomes tels que l'oxygène et/ou un halogène, ainsi que leurs mélanges.

7. Procédé selon la revendication 6, dans lequel le solvant est choisi parmi les hydrocarbures aromatiques alkylés portant un ou plusieurs groupes alkyles contenant de 1 à 4 atomes de carbone, les hydrocarbures aliphatiques saturés contenant de 5 à 12 atomes de carbone et les hydrocarbures aliphatiques cycliques.

8. Procédé selon la revendication 7, dans lequel le solvant est le toluène, le xylène, le mésitylène, l'éthylbenzène ou le butylbenzène, de préférence le xylène.

9. Procédé selon l'une quelconque des revendications 1 à 8, dans lequel on soumet le pied de distillation, qui contient un mélange de solvant et de sous-produits, à une évaporation, éventuellement sous vide, afin de récupérer le solvant à l'état épuré, et de le recycler à la distillation.

10. Procédé selon l'une quelconque des revendications 1 à 9 dans lequel l'oxirane est le 1,2-époxy-3-chloropropane ou le 1,2-époxypropane, et le composé oléfinique est le chlorure d'allyle ou le propylène.

## Patentansprüche

1. Verfahren zur Herstellung eines Oxirans durch Reaktion zwischen einer Olefinverbindung und Wasserstoffperoxid in einem wasserhältigen flüssigen Milieu, wonach ein Gemisch von Reaktionsprodukten gewonnen wird, das das Oxiran, Wasser und durch Öffnen des Oxiranringes gebildete hydroxylierte Nebenprodukte umfaßt, wenigstens ein Teil des Oxirans vom Gemisch der Reaktionsprodukte abgetrennt wird, ein das Wasser und die Nebenprodukte enthaltender Abstrom gewonnen wird, dem Abstrom ein organisches Lösungsmittel zugesetzt wird und das den Abstrom und das Lösungsmittel enthaltende Gemisch einer Destillationsbehandlung unterworfen wird, wobei das organische Lösungsmittel zum Extrahieren der Nebenprodukte aus dem wäßrigen Abstrom befähigt ist und eine sehr geringe Mischbarkeit mit Wasser aufweist, sodaß sich am Destillationskopf zwei flüssige Phasen ausbilden.

2. Verfahren nach Anspruch 1, worin die Nebenprodukte zusätzlich eine oder mehrere halogenierte Gruppen enthalten.

3. Verfahren nach Anspruch 1 oder 2, worin nach der Destillation einerseits am Destillationskopf eine erste flüssige Phase gewonnen wird, die das Lösungsmittel enthält, und eine zweite flüssige Phase gewonnen wird, die das von Nebenprodukten gereinigte Wasser und gegebenenfalls Lösungsmittelspuren enthält, und anderseits am Destillationssumpf ein Gemisch von Lösungsmittel und Nebenprodukten gewonnen wird, die am Destillationskopf gewonnenen beiden flüssigen Phasen getrennt werden und die das Lösungsmittel enthaltende erste flüssige Phase zur Destillation zurückgeführt wird.

4. Verfahren nach Anspruch 3, worin das spezifische Gewicht des Lösungsmittels sich um wenigstens 0,04 g/cm³ von demjenigen der flüssigen Phase unterscheidet, die das gereinigte Wasser enthält und am Destillationskopf gewonnen wurde.

5. Verfahren nach einem der Ansprüche 1 bis 4, worin der Siedepunkt des Lösungsmittels sich um wenigstens 5°C von demjenigen der Nebenprodukte unterscheidet.

6. Verfahren nach einem der Ansprüche 1 bis 5, worin das Lösungsmittel unter den aliphatischen oder aromatischen organischen Derivaten, die solche Atome wie Sauerstoff und/oder ein Halogen einschließen können, sowie unter deren Gemischen ausgewählt wird.

7. Verfahren nach Anspruch 6, worin das Lösungsmittel unter den alkylierten aromatischen Kohlenwasserstoffen, die eine oder mehrere Alkylgruppen mit 1 bis 4 Kohlenstoffatomen tragen, unter den gesättigten aliphatischen Kohlenwasserstoffen mit einem Gehalt an 5 bis 12 Kohlenstoffatomen und unter den cyclischen aliphatischen Kohlenwasserstoffen ausgewählt wird.

8. Verfahren nach Anspruch 7, worin das Lösungsmittel Toluol, Xylol, Mesitylen, Ethylbenzol oder Butylbenzol ist, vorzugsweise Xylol ist.

9. Verfahren nach einem der Ansprüche 1 bis 8, worin der Destillationssumpf, der ein Gemisch von Lösungsmittel und Nebenprodukten enthält, einem Verdampfen unterzogen wird, gegebenenfalls im Vakuum, um das Lösungsmittel im gereinigten Zustand zurückzugewinnen und es zur Destillation zurückzuführen.

10. Verfahren nach einem der Ansprüche 1 bis 9, worin das Oxiran das 1,2-Epoxy-3-chlorpropan oder das 1,2-Epoxypropan ist und die Olefinverbindung das Allylchlorid oder das Propylen ist.

## Claims

1. Process for the manufacture of an oxirane by reaction between an olefinic compound and hydrogen peroxide in a water-containing liquid medium, according to which a mixture of reaction products comprising the oxirane, water and hydroxylated by-products formed by opening the ring of the oxirane is collected, at least a portion of the oxirane is separated from the mixture of reaction products, an effluent containing water and by-products is collected, an organic solvent is added to the effluent ant the mixture containing the effluent and the solvent is subjected to a distillation treatment, the organic solvent being capable to extract the by-products from the aqueous effluent, and presenting a very low miscibility with water such that two liquid phases are formed at the distillation front.

2. Process according to Claim 1, in which the by-products contain one or more halogenated groups.

3. Process according to Claim 1 or 2, in which there are collected after distillation, on the one hand, at the distillation front, a first liquid phase containing the solvent and a second liquid phase containing water purified with respect to the by-products and possible traces of solvent, and on the other hand, at the distillation base, a mixture of solvent and by-products, the two liquid phases collected at the distillation front are separated, and the first liquid phase containing the solvent is recycled into the distillation.

4. Process according to Claim 3, in which the specific gravity of the solvent differs from that of the liquid phase containing the purified water collected at the distillation front by at least 0.04 g/cm³.

5. Process according to any one of Claims 1 to 4, in which the boiling point of the solvent differs from that of the by-products by at least 5°C.

6. Process according to any one of Claims 1 to 5, in which the solvent is chosen from the aliphatic or aromatic organic derivatives which may include atoms such as oxygen and/or a halogen, as well as mixtures thereof.

7. Process according to Claim 6, in which the solvent is chosen from alkylated aromatic hydrocarbons carrying one or more alkyl groups containing from 1 to 4 carbon atoms, saturated aliphatic hydrocarbons containing 5 to 12 carbon atoms and cyclic aliphatic hydrocarbons.

8. Process according to Claim 7, in which the solvent is toluene, xylene, mesitylene, ethylbenzene or butylbenzene, preferably xylene.

9. Process according to any one of Claims 1 to 8, in which the distillation base, which contains a mixture of solvent and by-products, is subjected to evaporation, optionally under vacuum, in order to recover the solvent in the purified state, and to recycle it into the distillation.

10. Process according to any one of Claims 1 to 9 in which the oxirane is 1,2-epoxy-3-chloropropane or 1,2-epoxypropane, the olefin compound is allyl chloride or propylene.
